# EUROPÄISCHE PATENTSCHRIFT

(11) **EP 2 987 480 B1**
(45) Veröffentlichungstag und Bekanntmachung des Hinweises auf die Patenterteilung: **19.06.2019**
(21) Anmeldenummer: 14181463.2
(22) Anmeldetag: 19.08.2014
(51) Int. Cl.: A61K 6/00

(54) **Lichthärtende Dentalkomposite mit zunehmender Opazität**
Photocurable dental composites with increasing opacity
Composite dentaire durcissant à la lumière présentant une opacité croissante

(43) Veröffentlichungstag der Anmeldung: 24.02.2016
(73) Patentinhaber: Ivoclar Vivadent AG, 9494 Schaan (LI)
(72) Erfinder: Vogel, Karin, 9487 Gamprin (LI); Tauscher, Sven, 8934 Knonau (CH); Moszner, Norbert, 9495 Triesen (LI)
(74) Vertreter: Uexküll & Stolberg

(56) Entgegenhaltungen:
- EP-A1- 2 649 981
- DE-A1-102004 017 124
- DE-U1-202008 018 436
- US-A1- 2006 178 469
- US-A1- 2008 076 847
- US-A1- 2009 298 966
- US-A1- 2013 203 884

## Beschreibung

Die vorliegende Erfindung betrifft lichthärtende Komposite zur Anwendung als dentale Zemente und Füllungskomposite.

Dentalmaterialien, die z.B. als Zement oder als direktes Füllungsmaterial, eingesetzt werden, enthalten in der Regel eine polymerisierbare organische Matrix und einen oder mehrere Füllstoffe, die meist mit einem polymerisationsfähigen Haftvermittler oberflächenmodifiziert sind. Der Füllstoffgehalt hängt maßgeblich vom gewünschten Verwendungszweck ab und kann bis zu 90 Gew.-% betragen, wobei Befestigungszemente im Vergleich zu Füllungsmaterialien einen geringeren Füllungsgrad aufweisen. Die polymerisierbare organische Matrix enthält gewöhnlich eine Mischung von Harzmonomeren, Initiatorkomponenten, Stabilisatoren und Pigmenten. Dentalmaterialien, die eine polymerisierbare Matrix und Füllstoff enthalten, werden als Komposite bezeichnet. Die polymerisierbare Matrix wird auch als Harz bezeichnet.

Als Monomere werden meist Mischungen von Dimethacrylaten eingesetzt. Verbreitete Beispiele hierfür sind die hochviskosen Dimethacrylate 2,2-Bis[4-(2-hydroxy-3-methacryloyloxypropyl)-phenyl]propan (Bis-GMA) und 1,6-Bis-[2-methacryloyloxyethoxycarbonylamino]-2,2,4-trimethylhexan (UDMA) und die als Verdünnermonomere genutzten niedrigerviskosen Dimethacrylate Bis(3-methacryloyloxymethyl)tricyclo-[5.2.1.0^{2,6}]decan (TCP), Decandiol-1,10-dimethacrylat (D₃MA) und Triethylenglycoldimethacrylat (TEGDMA). Dimethacrylate bewirken bei der Polymerisation eine dreidimensionale Vernetzung der sich bildenden Polymerketten und ergeben damit eine verbesserte mechanische Stabilität.

Die Materialen enthalten gewöhnlich auch einen Initiator für die radikalische Polymerisation, wobei lichthärtende Werkstoffe, die einen Photoinitiator enthalten, in der zahnärztlichen Füllungstherapie heutzutage eine dominante Stellung einnehmen.

Ein Nachteil von lichthärtenden Materialien ist, dass insbesondere das Legen großer Füllungen mit einem erheblichen Aufwand verbunden ist, weil das zur Härtung erforderliche Licht nur bis zu einer begrenzten Tiefe in die Materialien eindringen kann. Bei der sogenannten Inkrementtechnik wird die Füllung daher schichtweise aus dem Kompositwerkstoff aufgebaut, wobei die Schichten eine Dicke von jeweils ca. 2 mm haben und einzeln gehärtet werden.

Neuerdings finden sogenannte "Bulk-Fill"-Komposite, die Schichtdicken von 4 bis 5 mm erlauben, aufgrund der möglichen Zeitersparnis großes Interesse. Voraussetzung für die klinische Eignung dieser Materialien ist eine große Durchhärtungstiefe. Diese korreliert unter anderem mit der Transluzenz der Werkstoffe, wobei die Transluzenz maßgeblich durch die Brechungsindices der Harzmatrix und der Füllstoffe beeinflusst wird. In Kompositen kann eine hohe Transluzenz und damit eine gute Durchhärtungstiefe dann erreicht werden, wenn die organische Matrix und die verwendeten Füllstoffe übereinstimmende Brechungsindices aufweisen.

Gegenwärtig eingesetzte Bulk-Fill-Füllungskomposite zeichnen sich durch eine hohe Transluzenz vor und nach der Aushärtung aus. Dies ist im Hinblick auf die Durchhärtungstiefe ein Vorteil, nachteilig daran ist aber, dass die Komposite aufgrund der hohen Transluzenz das darunterliegende Dentin schlecht abdecken, was aus ästhetischen Gründen unerwünscht ist, weil die Farbe des Dentins von der des sichtbaren Zahnschmelzes abweicht.

Ein weiteres Problem ist die sich bei der Polymerisation aufbauende, mit zunehmender Schichtdicke steigende Polymerisationsschrumpfungsspannung (PKS). Hinzu kommt erschwerend, dass die PKS bei der Lichthärtung besonders hoch ist (Braga et al., Dent. Mater. 21 (2005) 962-970).

Der Erfindung liegt die Aufgabe zugrunde, lichthärtende Dentalwerkstoffe mit großer Durchhärtungstiefe bereitzustellen, die nach der Aushärtung eine hohe Abdeckgraft aufweisen, eine mit der natürlichen Zahnsubstanz vergleichbare Transluzenz haben und die sich auch zur Restauration von Zähnen im sichtbaren Bereich des Mundes eignen. Außerdem sollen sich die Werkstoffe in kurzer Zeit härten lassen und einen möglichst geringen Polymerisationsschrumpf und eine geringe PKS aufweisen.

Die Aufgabe wird erfindungsgemäß durch radikalisch polymerisierbare Dentalwerkstoffe gelöst, die
(1) 5 bis 40 Gew.-% mindestens eines polyfunktionellen radikalisch polymerisierbaren Monomers (A),
(2) 2 bis 15 Gew.-% mindestens eines monofunktionellen radikalisch polymerisierbaren Monomers (B),
(3) 0,1 bis 3,0 Gew.-% mindestens eines Photoinitiators für die radikalische Polymerisation (C) und
(4) 10 bis 85 Gew.-% Füllstoff(e) enthalten.

Die Werkstoffe sind dadurch gekennzeichnet, dass der Dentalwerkstoff einen Füllstoff (D) enthält und die Mischung der Monomere (A) und (B) einen Brechungsindex n_{D} von 1,50 bis 1,70, vorzugweise 1,50 bis 1,60, aufweist und dass der Brechungsindex der Monomermischung vor der Härtung dem Brechungsindex des Füllstoffs (D) entspricht oder um bis zu 0,013 größer ist und nach der Härtung um mindestens 0,02 größer als der Brechungsindex des Füllstoffs (D) ist. Die Werkstoffe haben im ungehärteten Zustand vorzugsweise die Form einer Paste. Die Mischung der Monomere (A) und (B) wird im Folgenden auch als Matrix bezeichnet.

Der Werkstoff kann als Füllstoff (D) eine Füllstoffmischung enthalten, wobei zu Füllstoff (D) alle Füllstoffe gerechnet werden, deren Brechungsindex in dem Bereich von n_{D}^{Matrix} - n_{D}^{Füller(D)} = 0 bis 0,013 liegt (n_{D}^{Matrix} = Brechungsindex der ungehärteten Monomermischung).

Die erfindungsgemäßen Dentalwerkstoffe weisen vor der Polymerisation eine hohe Transluzenz auf, weil die Brechungsindices von Monomeren und Füllstoff nur wenig voneinander abweichen. Das zur Polymerisation eingesetzte Licht kann daher tief in die Materialien eindringen, was eine große Durchhärtungstiefe gewährleistet. Bei der Polymerisation nimmt der Brechungsindex der Monomeren zu, während der Brechungsindex des oder der Füllstoffe unverändert bleibt. Dadurch vergrößert sich die Differenz zwischen den Brechungsindices von Monomeren und Füllstoff, und die Transluzenz nimmt dementsprechend ab. Die Monomeren werden so gewählt, dass der Brechungsindex der Monomermischung vor der Härtung um 0 bis 0,13 größer als der Brechungsindex des Füllstoffs ist und nach der Härtung um mindestens 0,02, vorzugsweise um 0,020 bis 0,045.

Der Brechungsindex des natürlichen Zahnschmelzes liegt bei ca. 1,62-1,66, und der Brechungsindex des unter dem Zahnschmelz liegenden Dentins bei ca. 1,45. Die Transluzenz des Zahnes ergibt sich aus dem Gesamteindruck der hoch transparenten, dünnen Schmelzschicht und des Dentins. Die erfindungsgemäßen Werkstoffe weisen nach der Härtung vorzugsweise eine Transluzenz von 5 bis 15 % auf. Es wurde gefunden, dass solche Materialien dem Erscheinungsbild der natürlichen Zahnsubstanz sehr nahe kommen und gleichzeitig eine optisch zufriedenstellende Abdeckung des Dentins ermöglichen. Die Materialien erlauben damit eine ästhetisch zufriedenstellende Behandlung von Zahndefekten auch im sichtbaren Bereich des Mundes eines Patienten. Die Transluzenz der Werkstoffe kann wie unten genauer beschrieben mit einem Spektralphotometer bestimmt werden.

Bei der Herstellung radikalisch polymerisierbarer Dentalwerkstoffe werden zur Gewährleistung einer hohen mechanischen Belastbarkeit primär polyfunktionelle Monomere eingesetzt. Diese haben aber den Nachteil, dass durch die Ausbildung eines dreidimensionalen Netzwerks die Viskosität rasch ansteigt, so dass der Polymerisationsschrumpf nicht mehr durch viskoses Fließen des Materials ausgeglichen werden kann. Dies hat eine hohe Polymerisationsschrumpfungsspannung (PKS) zur Folge. Andererseits ist eine hohe Viskosität im Hinblick auf die Geschwindigkeit der radikalischen Reaktion vorteilhaft, weil eine hohe Viskosität eine schnelle Aushärtung der Materialien begünstigt. Die Verringerung der PKS und die gleichzeitige Erzielung einer hohen Reaktionsgeschwindigkeit sind daher schwierig.

Bei der Härtung von Dentalwerkstoffen kommt weiterhin der Volumenkontraktion bei der Polymerisation eine entscheidende Bedeutung zu, weil der Polymerisationsschrumpf zur Randspaltbildung bei Füllungskompositen führen kann. Allerdings zeigen gerade Monomere mit hoher Volumenkontraktion eine große Zunahme des Brechungsindex bei der Polymerisation, was im Hinblick auf die angestrebte Transluzenzabnahme vorteilhaft ist.

Erfindungsgemäß wurde gefunden, dass durch die Kombination von Monomeren und die Abstimmung des Brechungsindex der Monomermischung auf den des Füllstoffs ein idealer Kompromiss hinsichtlich der genannten Parameter erzielt werden kann. Die Werkstoffe weisen in ungehärtetem Zustand eine hohe Transluzenz auf und erlauben so auch die Härtung dicker Schichten. Bei der Härtung nimmt die Transluzenz ab, so dass die Eigenfarbe des Dentins abgedeckt und eine optische Anpassung an den Zahnschmelz erzielt wird.

Die erfindungsgemäßen Werkstoffe enthalten als Komponente (B) mindestens ein monofunktionelles Monomer. Monofunktionelle Monomere beeinflussen die Netzwerkbildung. Unter monofunktionellen Monomeren werden Verbindungen mit einer, unter polyfunktionellen Monomeren Verbindungen mit zwei oder mehr, vorzugsweise 2 bis 4 und insbesondere 2 radikalisch polymerisierbaren Gruppen verstanden.

Als monofunktionelle Monomere lassen sich z.B. N-monosubstituierte Acrylamide, wie z.B. N-Ethylacrylamid einsetzen. Erfindungsgemäß bevorzugte monofunktionelle Monomere sind Monomethacrylate. Besonders bevorzugte monofunktionelle Methacrylate sind Methyl-, Ethyl-, 2-Hydroxyethyl-, Butyl-, Benzyl-, Tetrahydrofurfuryl- und Isobornylmethacrylat, p-Cumyl-phenoxyethylenglycolmethacrylat (CMP-1E) und Mischungen davon, ganz besonders bevorzugt sind Benzyl-, Tetrahydrofurfuryl- oder Isobornylmethacrylat, CMP-1E und Mischungen davon. Am meisten bevorzugt ist CMP-1E.

Als polyfunktionelle Monomere (A) lassen sich z.B. N- disubstitiuierte Acrylamide, wie z.B. N,N-Dimethylacrylamid, und Bisacrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)piperazin einsetzen. Erfindungsgemäß sind polyfunktionelle und insbesondere difunktionelle Methacrylate bevorzugt, wie z.B. 1,6-Bis-[2-methacryloyloxyethoxycarbonylamino]-2,2,4-trimethylhexan (UDMA; ein Additionsprodukt aus 2-Hydroxyethylmethacrylat und 2,2,4-Trimethylhexamethylendiisocyanat), Di-, Tri- oder Tetraethylenglycoldimethacrylat, Trimethylolpropantrimethacrylat, Pentaerythrittetramethacrylat, sowie Glycerindi- und -trimethacrylat, 1,4-Butandioldimethacrylat, 1,10-Decandioldimethacrylat (D₃MA), 1,12-Dodecandioldimethacrylat und Mischungen davon.

Besonders bevorzugte Dimethacrylate sind Bisphenol-A-dimethacrylat, 2,2-Bis[4-(2-hydroxy-3-methacryloyloxypropyl)-phenyl]propan (Bis-GMA; ein Additionsprodukt aus Methacrylsäure und Bisphenol-A-diglycidylether), ethoxy- oder propoxyliertes Bisphenol-A-Dimethacrylat, wie z.B. das Bisphenol-A-Dimethacrylat 2-[4-(2-Methacryloyloxyethoxyethoxy)phenyl]-2-[4-(2-methacryloyloxyethoxy)phenyl]propan) (SR-348c, Firma Sartomer), und 2,2-Bis[4-(2-methacryloxypropoxy)phenyl]propan, Bis-(3-methacryloyloxymethyl)tricyclo-[5.2.1.0^{2,6}]decan (TCP) und Mischungen davon. Ganz besonders bevorzugte polyfunktionelle Monomere sind 2,2-Bis[4-(2-methacryloxypropoxy)phenyl]propan, Bis-GMA, SR-348c, TCP und Mischungen davon.

Erfindungsgemäß werden Monomere und Monomermischungen eingesetzt, die im ungehärteten Zustand einen Brechungsindex von 1,50 bis 1,70, vorzugsweise 1,50 bis 1,60 aufweisen, wobei im Fall von Monomermischungen solche Mischungen besonders bevorzugt sind, die ausschließlich Monomere mit einem Brechungsindex von 1,50 bis 1,70 und insbesondere 1,50 bis 1,60 enthalten. Bei der Kombination mit den erfindungsgemäß verwendeten Füllstoffen werden Kompositpasten mit hoher Transluzenz erhalten. Die bevorzugten Monomere zeigen bei ihrer Polymerisation eine große Änderung des Brechungsindexes, was zu einer deutlichen Abnahme der Transluzenz führt, wobei Monomere mit einem Polymerisationsschrumpf von weniger als 10 Vol.-%, insbesondere von 5 bis 9 Vol.-% bevorzugt sind. Wenn nicht anders angegeben wird der Polymerisationsschrumpf hierin nach der ISO-Norm 17304:2013 bestimmt.

Der Brechungsindex von Monomeren ist umso grösser, je mehr Elektronen ein Molekül enthält und je polarisierbarer diese Elektronen sind. Der Brechungsindex von Polymeren nimmt mit der Dichte und mit steigender Kristallinität zu und liegt bei organischen Polymeren zwischen 1,29-1,73. (vgl. H.-G. Elias, Makromoleküle - Anwendungen von Polymeren, Bd. 4. 6. Auflage, Wiley-VCH, Weinheim 2003, 513-515). Rein aliphatische oder cycloaliphatische Methacrylate haben meist einen Brechungsindex von unter 1,50, sofern keine Elemente mit höherer Ordnungszahl, z.B. Schwefel oder Brom, enthalten sind. UDMA hat beispielsweise einen Brechungsindex (n_{D}) von 1,485 (Polymer 1,510) und TEGDMA von 1,461 (Polymer 1,508). Dagegen zeigen aromatische Monomere n_{D}-Werte über 1,50 und sind daher als Komponente (A) und (B) bevorzugt. Beispielsweise ist der Brechungsindex von propoxyliertem Bis-GMA 1,54 und der von CMPE-1E 1,5525 (Polymer 1,5793). Durch den Einbau von schweren Elementen wie z.B. Schwefel, Brom oder Iod kann der Brechungsindex weiter erhöht werden. Durch das Mischen von Monomeren mit unterschiedlichen Brechungsindices kann der Brechungsindex der Monomermischung an den Brechungsindex der verwendeten Füllstoffe angepasst werden.

Weiterhin sind solche Monomermischungen bevorzugt, die mindestens ein schwerflüchtiges Monomethacrylat, vorzugsweise in einer Menge von 0-30 Gew.-%, besonders bevorzugt 5-30 Gew.-%, ganz besonders bevorzugt 10-25 Gew.-%, mindestens ein hochviskoses difunktionelles Methacrylat, vorzugsweise in einer Menge von 5-50 Gew.-%, besonders bevorzugt 10-35%, und mindestens ein niedrigviskoses Dimethacrylat, vorzugsweise in einer Menge von 5-30 Gew.-%, besonders bevorzugt 8-25%, enthalten. Diese Prozentangaben beziehen sich auf die Gesamtmasse der Monomermischung. Monomermischungen, die ausschließlich die genannten Monomere enthalten, sind besonders bevorzugt. Auch hier sind in allen Fällen Monomere mit einem Brechungsindex von 1,50 bis 1,70 und/oder einem Polymerisationsschrumpf (ΔV_{P}) unter 10 Vol.-%, insbesondere 5-9 Vol.-%, bevorzugt.

Erfindungsgemäß werden unter schwerflüchtigen Monomeren Verbindungen mit einem Siedepunkt > 150 °C bei Normaldruck verstanden. Der Siedepunkt kann z.B. mit einer Destillationsapparatur bestimmt werden. Unter hochviskosen Monomeren werden Stoffe mit einer Viskosität ≥ 5 Pa·s, vorzugsweise 5 bis 10.000 Pa·s und besonders bevorzugt 5 bis 2.000 Pa·s, und unter niedrigviskosen Monomeren Stoffe mit einer Viskosität ≤ 3 Pa·s, vorzugsweise 100 bis 3.000 mPa·s und besonders bevorzugt 500 bis 2.000 mPa·s, verstanden, wobei die Viskosität mit einem Kapillar- (niedrigviskos) oder Rotationsviskosimeter (hochviskos) bei einer Temperatur von 25°C bestimmt wird.

Ganz besonders bevorzugt sind Mischungen aus dem Monomethacrylat CMP-1E (ΔV_{P} = 7,3 Vol.-%, n_{D} = 1,5525) mit dem hochviskosen Bis-GMA (η = ca. 800 Pa·s, ΔV_{P} = 6,1 Vol.-%, n_{D} = 1,549) und den niedrigviskosen Monomeren SR-348c (η = 550-1700 mPa·s, ΔV_{P} = 5,9 Vol.-%, n_{D} = 1,536) und/oder TCP (η = ca. 1200 mPa·s, ΔV_{P} = 6,4 Vol.-%, n_{D} =1,501). Diese Mischungen zeichnen sich durch eine besonders geringe Zytotoxizität aus.

Die erfindungsgemäßen Werkstoffe können außerdem auch ein oder mehrere acide Monomere enthalten. Als acide Monomere eignen sich besonders polymerisationsfähige Carbonsäuren, wie 4-(Meth)acryloyloxyethyltrimellitsäure; Phosphonsäuremonomere, wie 2-Methacryloyloxyethylphosphonsäure, 2-Methacrylamidoethylphosphonsäure, 4-Meth-acrylamido-4-methyl-pentyl-phosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester; sowie polymerisationsfähige Phosphorsäureester, wie 2-Methacryloyloxyethyldihydrogenphosphat, 10-Methacryloyloxyde¬icyl-dihydrogenphosphat, 6-(Methacryl-amido)hexyldihydrogenphosphat und 1,3-Bis-(N-acryloyl-N-propyl-amino)-propan-2-yl-dihydrogenphosphat. Besonders geeignete acide Monomere sind 4-(Meth)acryloyloxyethyltrimellitsäure, 2-[4-(Dihydroxyphospho¬ryl)-2-oxa-butyl]-acrylsäureethyl- oder -2,4,6-trimethylphenylester und 10-Methacryl¬oyloxydecyl-dihydrogenphosphat. Die aciden Monomere verbessern die Haftung der Werkstoffe am Zahn und werden daher vor allem zur Herstellung selbsthaftender Kompositzemente eingesetzt. Der Gehalt an aciden Monomeren liegt vorzugsweise im Bereich von 0-15 Gew.-%, besonders bevorzugt 1-15 Gew.-% (bezogen auf die Gesamtmasse an Monomeren).

Der Brechungsindex ist eine Stoffkonstante, die von der Wellenlänge des verwendeten Lichts, von der Temperatur, vom Druck und der Reinheit des Stoffes abhängt. Wenn nicht anders angegeben wird hier unter dem Brechungsindex der bei 20°C mit dem Licht der gelben Na-D-Linie (λ = 589 nm) gemessene Brechungsindex verstanden (n_{D}²⁰ oder kurz n_{D}). Die Bestimmung des Brechungsindex flüssiger Monomere und Monomermischungen kann mit einem handelsüblichen Abbe-Refraktometer erfolgen.

Die Bestimmung des Brechungsindex von festen Stoffen wie z.B. von anorganischen Füllstoff- oder Polymerpulvern erfolgt nach der Immersionsmethode. Die Stoffe werden bei 20°C in Flüssigkeiten mit unterschiedlichen Brechungsindices dispergiert (sogenannte Immersionsflüssigkeiten). Dabei zeigen sich die Konturen der Feststoffpartikel umso deutlicher, je größer der Brechzahlunterschied zwischen Flüssigkeit und Feststoff ist. Ändert man nun die Brechzahl der Flüssigkeit so, dass sie der des Feststoffs näher kommt, werden die Partikelkonturen schwächer und verschwinden bei Angleichung der Brechindices völlig. Als Immersionsflüssigkeiten eignen sich Flüssigkeiten mit bekanntem Brechungsindex, z.B. Mischungen aus Benzylsalicylat (n_{D}²⁰ = 1,536) und Triacetin (n_{D}²⁰ = 1,431) oder Bromnaphthalin (n_{D}²⁰ = 1,657). Durch die Variation der Mengenverhältnisse dieser Stoffe kann der Brechungsindex der Mischung an den des zu messenden Feststoffs angepasst werden. Bei Übereinstimmung der Brechungsindices wird der Brechungsindex der Immersionsflüssigkeit mit einen Refraktometer bestimmt.

Die Übereinstimmung der Brechungsindices von Feststoff und Immersionsflüssigkeit kann durch Betrachtung der Becke'schen Linie ermittelt werden (Becke-Linien-Verfahren). Dabei handelt es sich um einen hellen Lichtsaum, der sich beim Defokussieren einer Grenzfläche zeigt. Der zu prüfende Feststoff wird in eine Flüssigkeit mit bekanntem Brechungsindex gegeben und im Mikroskop mit monochromatischem Licht beobachtet. Besitzen Probekörper und Flüssigkeit unterschiedliche Brechungsindices, zeigt sich um jedes Partikel ein schmaler, leuchtender Ring (Becke-Linie), der sich beim Fokussieren bewegt. Dieser Vorgang wird in verschiedenen Flüssigkeiten mit anderen Brechungsindices so lange wiederholt, bis keine Becke-Linien mehr auftreten und somit der Brechungsindex von Probekörper und Flüssigkeit übereinstimmen.

Gemäß einer ersten Ausführungsform enthalten die erfindungsgemäßen Werkstoffe ausschließlich Füllstoff(e) (D), wobei die Brechungsindices von Füllstoff und Matrix wie oben beschrieben definiert sind, und ggf. solche Füllstoffe, die sichtbares Licht nicht streuen und die daher die Transluzenz nicht beeinflussen. Andere Füllstoffe sind nicht vorhanden.

Gemäß einer zweiten Ausführungsform enthalten die erfindungsgemäßen Werkstoffe neben dem Füllstoff (D) zusätzlich einen oder mehrere Füllstoffe (E) mit einem Brechungsindex, der um 0,02 bis maximal 0,155, vorzugsweise 0,03 bis 0,055 kleiner als der Brechungsindex der Matrix ist. Der Füllstoff (E) weist damit einen kleineren Brechungsindex als der Füllstoff (D) auf. Als Füllstoff (E) kann ebenfalls eine Füllstoffmischung verwendet werden, wobei alle Füllstoffe, deren Brechungsindex im Bereich von n_{D}^{Matrix} - n_{D}^{Füller(E)} = 0,02 bis 0,155, vorzugweise 0,03 bis 0,055 liegt, zu Füllstoff (E) gerechnet werden.

Es wurde gefunden, dass durch eine Kombination von Füllstoff (D) und Füllstoff (E) bei gleicher Füllstoffmenge die Abnahme der Transluzenz bei der Polymerisation verstärkt werden kann. Die Abnahme der Transluzenz bei der Polymerisation hängt von der Füllstoffmenge und von der Zunahme der Differenz der Brechungsindices ab. Bei höheren Füllstoffmengen oder einer größeren Zunahme der Differenzen fällt die Transluzenz stärker ab. Die Zugabe des Füllstoffs (E) eröffnet eine weitere Möglichkeit zur Steuerung der Transluzenzabnahme. Der Effekt des Füllstoffs (E) ist umso größer je tiefer sein Brechungsindex im Vergleich zum Hauptfüllers ist.

Da der Brechungsindex des Füllers (E) kleiner als der des Füllers (D) ist, ist die Abweichung vom Brechungsindex der ungehärteten Monomermischung größer, so dass der Füllstoff (E) zu einer Verringerung der Transluzenz der ungehärteten Werkstoffe führen kann. Aus diesem Grunde beträgt der Anteil des Füllstoffs (E) an der gesamten Füllstoffmenge maximal 15 Gew.-%. Die Menge des Füllstoffs (E) liegt in einem Bereich von 0 bis 15 Gew.-%, bevorzugt 0 bis 10 Gew.-%, besonders bevorzugt 0 bis 5 Gew.-% und insbesondere 1 bis 5 Gew.-% bezogen auf die Gesamtfüllstoffmenge.

Gemäß der zweiten Ausführungsform sind erfindungsgemäß solche Werkstoffe bevorzugt, die ausschließlich Füllstoff(e) (D) und (E) und ggf. solche Füllstoffe enthalten, die sichtbares Licht nicht streuen und die daher die Transluzenz nicht beeinflussen. Andere Füllstoffe sind nicht vorhanden.

Neben dem Füllstoff (D) und ggf. dem Füllstoff (E) sowie Füllstoffen, die sichtbares Licht nicht streuen und die daher die Transluzenz nicht beeinflussen, können die erfindungsgemäßen Werkstoffe gemäß einer weiteren Ausführungsform auch einen untergeordneten Anteil an Füllstoff enthalten, der in keine dieser Kategorien fällt (Füllstoff F).

Als Füllstoff (F) werden primär solche Füllstoffe eingesetzt, die zur gezielten Beeinflussung bestimmter Eigenschaften der Werkstoffe dienen, wie z.B. der Röntgenopazität. Sie werden nur in der Menge zugesetzt, der zur Erzielung der gewünschten Wirkung erforderlich ist.

Solche Füllstoffe können sich nachteilig auf die Abnahme der Transluzenz beim Härten auswirken. Wenn der Brechungsindex des Füllers (F) größer als der der Matrix ist, nähert sich der Brechungsindex der Monomermischung, anders als im Fall der Füllstoffe (D) und (E), bei der Polymerisation dem des Füllstoffs an, so dass die Differenz der Brechungsindices kleiner wird. Der Füller (F) neutralisiert in diesem Fall den Effekt der Füllstoffe (D) und (E). Der Anteil des Füllers (F) an der gesamten Füllstoffmenge liegt daher im Bereich von 0 bis 15,5 Gew.-%, bevorzugt 0 bis 9,7 Gew.-% und besonders bevorzugt 0 bis 5,1 Gew.-%. Füllstoff (F) kann eine Mischung unterschiedlicher Füllstoffe sein. Die Gesamtmenge der Füllstoffe (E) und (F) beträgt vorzugsweise maximal 35 Gew.-% bezogen auf die gesamte Füllstoffmenge.

Der Brechungsindex des Füllstoffs (F) ist vorzugsweise um maximal 0,055 größer als der Brechungsindex der Matrix. Besonders bevorzugt liegt der Brechungsindex n_{D}^{Füller(F)} des Füllers (F) im Bereich (n_{D}^{Matrix} -0,02) < n_{D}^{Füller(F)} < (n_{D}^{Matrix} +0,055), wobei nur solcher Füllstoff zu Füllstoff (F) gerechnet wird, der nicht in eine der anderen Füllstoffkategorien fällt.

Die Menge an Füllstoff, der sichtbares Licht nicht streut und die Transluzenz nicht beeinflusst, liegt bei allen Ausführungsformen jeweils im Bereich von 0 bis 10 Gew.-%, bevorzugt 0 bis 1,8 Gew.-% bezogen auf die Gesamtfüllstoffmenge.

Die Füllstoffe (D), (E) und (F) werden jeweils aus den im Folgenden definierten Materialien ausgewählt. Die Zuordnung zu einer Füllstoffgruppe richtet sich nach dem Brechungsindex und dessen Differenz zum Brechungsindex der Matrix.

Die erfindungsgemäßen Dentalwerkstoffe können organische oder vorzugsweise anorganische oder organisch-anorganische Füllstoffe enthalten, wobei partikuläre Füllstoffe bevorzugt sind. Bevorzugte anorganische partikuläre Füllstoffe sind Pulver röntgenopaker Gläser mit einer durchschnittlichen Teilchengröße von 0,01 bis 15 µm, vorzugsweise 0,10 bis 5,0 µm; röntgenopake Füllstoffe, wie Ytterbiumtrifluorid, mit einer durchschnittlichen Teilchengröße von 0,050 bis 2,0 µm, vorzugsweise 0,10 bis 1,0 µm; Mischoxide aus SiO₂, ZrO₂, ZnO und/oder TiO₂ mit einer durchschnittlichen Teilchengröße von 5 bis 500 nm, vorzugsweise 20 bis 200 nm; nanopartikuläre Füllstoffe, wie Tantal(V)-oxid, Bariumsulfat oder Mischoxide von SiO₂ mit Ytterbium(III)-oxid oder Tantal(V)-oxid mit einer durchschnittlichen Teilchengröße von 5 bis 500 nm, vorzugsweise 20 bis 200 nm.

Unter organisch-anorganischen Füllstoffen werden Polymerpartikel verstanden, die ihrerseits mit anorganischen Füllstoffen gefüllt sind. Bevorzugt sind organisch-anorganische Füllstoffe mit einer durchschnittlichen Teilchengröße von 5 bis 100 µm, vorzugsweise von 10 bis 50 µm. Im Fall der organisch-anorganischen Füllstoffe wird der Brechungsindex der gehärteten Polymermatrix vorzugsweise so gewählt, dass er mit dem Brechungsindex des darin enthaltenen anorganischen Füllstoffs übereinstimmt, so dass die Füllstoffpartikel in der Matrix eine hohe Transluzenz aufweisen.

Organisch-anorganische Füllstoffe werden vorzugsweise durch thermische Härtung von Kompositpasten auf der Basis von Dimethacrylatmischungen und Füllstoffen hergestellt. Dabei kommen als Dimethacrylate bevorzugt Bis-GMA, UDMA und D₃MA zum Einsatz. Als Füllstoffe dienen vorzugsweise röntgenopake Glasfüllstoffe und/oder Ytterbiumtrifluorid. Der Brechungsindex der polymerisierten Monomermischung wird vorzugsweise so eingestellt, dass er dem Brechungsindex des verwendeten Hauptfüllstoffs entspricht. Die thermischen Polymerisate werden dann gemahlen und als Pulver eingesetzt.

Bei allen Partikelgrößen handelt es sich um Gewichtsmittel. Die Lichtstreuung nimmt mit abnehmender Partikelgröße ab, jedoch haben Füllstoffe mit geringer Partikelgröße eine größere Verdickungswirkung. Erfindungsgemäß sind daher Füllstoffe mit einer Partikelgröße im Bereich von 100 nm bis 5 µm und insbesondere im Bereich von 200 nm bis 2 µm bevorzugt.

Die röntgenopaken Gläser haben vorzugsweise einen Brechungsindex von 1,51-1,55, YbF₃ hat einen Brechungsindex von 1,545 und der Brechungsindex der übrigen Füllstoffe wie z.B. der organisch-anorganischen Füllstoffe liegt vorzugsweise im Bereich von 1,48 bis 1,54.

Neben den genannten Füllstoffen können die Werkstoffe Füllstoffe mit einer Partikelgröße von vorzugsweise < 50 nm, besonders bevorzugt < 40 nm enthalten. Die Partikelgröße liegt vorzugsweise im Bereich von 10-50 nm und besonders bevorzugt von 10-40 nm. Diese Füllstoffe streuen aufgrund ihrer geringen Partikelgröße sichtbares Licht nicht und haben damit keinen Einfluss auf die Transluzenz. Bevorzugte Beispiele für diese Füllstoffe sind pyrogene Kieselsäure und Fällungskieselsäure. Diese haben einen Brechungsindex von ca. 1,46.

Die Füllstoffe sind vorzugsweise oberflächenmodifiziert, besonders bevorzugt durch Silanisierung, insbesondere mit 3-Methacryloyloxypropyltrimethoxysilan. Die Silanisierung hat keinen messbaren Einfluss auf den Brechungsindex des Füllstoffs.

Die erfindungsgemäßen Dentalwerkstoffe zeigen bei der Polymerisation eine Abnahme der Transluzenz, die auf eine Zunahme der Differenz der Brechungsindices von polymerisierbarer Matrix und Füllstoff zurückzuführen ist. Dies erfordert eine Abstimmung der Brechungsindices von Monomer(en) und Füllstoff. Die Art, Menge und Brechungsindices des Monomers oder der Monomermischung und des Füllstoffs oder der Füllstoffe werden vorzugsweise so gewählt, dass der Werkstoff vor der Polymerisation eine Transluzenz von 15 bis 80%, vorzugsweise 20 bis 75% aufweist. Zur Bestimmung der Transluzenz gehärteter Werkstoffe werden Polymer- oder Kompositprüfkörper (runde Scheiben mit einem Durchmesser von 20 mm und einer Dicke von 1 mm) hergestellt und in einem Spektralphotometer farbmetrisch vermessen (Farbmessung in Transmission, Wellenlänge 360-740 nm). Bei ungehärteten Materialien. wird die Kompositpaste oder die Monomermischung in einer Glasküvette mit einer Schichtdicke von 1 mm farbmetrisch vermessen. Vorzugsweise wird zur Bestimmung der Transluzenz das Spectrophotometer CM-5 der Firma Minolta verwendet.

Die erfindungsgemäßen Dentalwerkstoffe enthalten vorzugsweise 20 bis 80 Gew.-%, besonders bevorzugt 40 bis 80 Gew.-% an röntgenopakem Glas oder Gläsern. Ytterbiumfluorid wird vorzugsweise in einer Menge von 0 bis 40 Gew.-%, besonders bevorzugt 0 bis 30 Gew.-% eingesetzt, Mischoxide vorzugsweise in einer Menge von 0 bis 70 Gew.-%, besonders bevorzugt 0 bis 40 Gew.-%. Organisch-anorganische Füllstoffe, sogenannte Komposit- oder Isofüller, werden vorzugsweise in einer Menge 0 bis 50 Gew.-% und besonders bevorzugt von 0 bis 30 Gew.-% eingesetzt. Ganz besonders bevorzugt sind Werkstoffe, die 49 bis 61 Gew.-% röntgenopakes Glas oder Gläser, 2,5 bis 6 Gew.-% YbF₃, 0 bis 10 Gew.-% Mischoxid(e) und 7 bis 17 Gew.-% Isofüller enthalten. Die Gesamtmenge aller Füllstoffe liegt vorzugsweise im Bereich von 5 bis 90 Gew.-%, besonders bevorzugt 10 bis 85 Gew.-% und ganz besonders bevorzugt 40 bis 80 Gew.-%. Alle Mengenangaben beziehen sich auf die Gesamtmasse des Werkstoffs.

Wenn Mischungen der genannten Füllstoffe eingesetzt werden, liegt das Gewichtsverhältnis von röntgenopaken Gläsern zu Ytterbiumfluorid vorzugsweise im Bereich von 10:0 bis 10:2, das Gewichtsverhältnis von röntgenopaken Gläsern zu Mischoxiden im Bereich von 10:0 bis 10:5 und das Gewichtsverhältnis von röntgenopaken Gläsern zu organisch-anorganischen Füllstoffen im Bereich von 10:0 bis 1:1. Besonders bevorzugt sind Füllstoffmischungen, die mindestens 50 Gew.-% mindestens eines röntgenopaken Glases enthalten, bezogen auf die Gesamtmasse an Füllstoffen.

Die erfindungsgemäßen Zusammensetzungen werden vorzugsweise durch Bestrahlen mit Blaulicht (Wellenlängenbereich von 400-500 nm) gehärtet, vorzugweise durch Bestrahlen mit einer LED-Lampe oder Halogenlampe. Hierzu enthalten die Werkstoffe vorzugsweise mindestens einen Photoinitiator, der in dem angegebenen Wellenlängenbereich aktiv ist.

Bevorzugte Photoinitiatoren sind Photosensibilisatoren, vor allem von α-Diketone, wie 9,10-Phenanthrenchinon, 1-Phenylpropan-1,2-dion, Diacetyl oder 4,4'-Dichlorbenzil oder deren Derivate, besonders bevorzugt Campherchinon (CC) und dessen Derivate, und Mischungen davon.

Die Photoinitiatoren werden vorzugsweise in Kombination mit Beschleunigern eingesetzt. Als Beschleuniger eignen sich besonders tertiäre Amine, wie z.B. tertiäre aromatische Amine, insbesondere N,N-Dialkyl-aniline, -p-toluidine oder -3,5-xylidine, p-(N,N-Dialkylamino-phenylethanol, -benzoesäurederivate, -benzaldehyd, -phenylessigsäureester und -phenylpropionsäureester. Konkrete Beispiele hierfür sind N,N-Dimethylanilin, N,N-Dimethyl-p-toluidin, N,N,3,5-Tetramethylanilin, N,N-Dimethylamino-p-benzaldehyd, p-(Dimethylamino)-benzoesäureethylester oder p-(Dimethylamino)-benzonitril. Geeignet sind auch tertiäre aliphatische Amine, wie z.B. Tri-n-butylamin, Dimethylaminoethan-2-ol, Triethanolamin, Dimethylaminoethylmethacrylat, N,N-Dimethylbenzylamin, oder heterocyclische Amine, wie z.B. 1,2,2,6,6-Pentamethylpiperidin, und Aminosäure-Derivate, wie z.B. N-Phenylglycin.

In Zusammensetzungen, die acide Monomere enthalten, wie z.B. selbsthaftende Komposite, werden vorzugsweise aminfreie Beschleuniger verwendet, wie z.B. Sulfinsäuren und Sulfinate, Borate, Enolate, Phosphine oder andere Verbindungen, die aktive Wasserstoffatome enthalten, z.B. heterocylische Verbindungen wie Morpholin-Derivate oder 1,3-Dioxolane.

Besonders bevorzugte Photoinitiatoren sind Acyl- oder Bisacylgermanium-Verbindungen, insbesondere die in der EP 1 905 413 A1 offenbarten Monoacyltrialkyl- und Bisacyldialkylgermanium-Verbindungen, wie z.B. Benzoyltrimethylgermanium, Bisbenzoyldiethylgermanium oder Bis(4-methoxybenzoyl)-diethylgermanium. Acyl- und Bisacylgermanium-Verbindungen haben den Vorteil, dass sie sich nach der Bestrahlung entfärben (Bleaching Effekt) und so die Transparenz der ausgehärteten Werkstoffe nicht beeinträchtigen. Außerdem handelt es sich um monomolekulare Photoinitiatoren, d.h. sie benötigen keinen Beschleuniger, um ihre volle Aktivität zu erreichen.

Weitere besonders bevorzugte Photoinitiatoren sind Acyl- oder Bisacylphosphinoxide, insbesondere die in EP 0 007 505, EP 0 073 413, EP 0 184 095 und EP 0 615 980 beschriebenen Verbindungen. Bevorzugte Beispiele sind die kommerziell zugänglichen Verbindungen 2,4,6-Trimethylbenzoyldiphenylphosphinoxid (Lucirin® TPO, BASF) und Bis(2,4,6-trimethylbenzoyl)phenylphosphinoxid (Irgacure® 819, Ciba). Acyl- und Bisacylphosphinoxide gehören ebenfalls zur Gruppe der monomolekularen Photoinitiatoren und zeichnen sich durch eine geringe Eigenabsorption aus.

Gegebenenfalls können die erfindungsgemäß eingesetzten Zusammensetzungen weitere Additive enthalten, vor allem Stabilisatoren, wie z.B. Polymerisationsstabilisatoren, Farbmittel, antibakterielle Wirkstoffe, fluoridionenabgebende Additive, optische Aufheller, Fluoreszenzmittel, UV-Absorber, Stoffe zur Verbesserung der Bruchzähigkeit und/oder Effektmittel.

Die erfindungsgemäßen Dentalwerkstoffe haben vorzugsweise eine Lichtempfindlichkeit von größer 60 s, besonders bevorzugt 80 s bis 300 s. Unter der Lichtempfindlichkeit wird die Zeit verstanden, während der die Werkstoffe ohne vorzeitige Härtung durch Umgebungslicht, wie z.B. der OP-Lampe des Zahnarztes, verarbeitet werden können. Die Lichtempfindlichkeit wird nach der ISO 4049: 2000: "Dentistry - Polymer-based filling, restorative and luting materials" bestimmt. Hierzu wird eine Werkstoffprobe mit einer Xenonlampe bei 8000 lx solange bestrahlt, bis die Polymerisation einsetzt. Die Lichtempfindlichkeit ist die Zeit in Sekunden bis zum Einsetzen der Polymerisation. Bei einer Lichtempfindlichkeit von 60 s beginnt der Werkstoff nach 60 s unter der Xenonlampe auszuhärten. Die Lichtempfindlichkeit kann durch die Zugabe von Polymerisationsinhibitoren, vorzugsweise von anaeroben Inhibitoren eingestellt werden.

Weiterhin haben die erfindungsgemäßen Dentalwerkstoffe vorzugsweise eine Röntgenopazität von 100% bis 500% Al, besonders bevorzugt 150% bis 300% Al. Die Röntgenopazität wird ebenfalls nach der oben genannten ISO-Norm 4949 bestimmt. Dabei wird ein Prüfkörper aus dem polymerisierten Dentalwerkstoff zusammen mit einer Aluminiumtreppe mit einer Stufenhöhe von 1 mm mit einer Röntgenkamera aufgenommen. Man vergleicht den Schwärzungsgrad der Bilder und gibt die Röntgenopazität in %Al an, 100% Röntgenopazität entsprechen dem Schwärzungsgrad von 1 mm Aluminium. Die Röntgenopazität kann durch die Menge an röntgenopaken Füllstoffen eingestellt werden, wie röntgenopaken Gläsern oder Ytterbium(III)-fluorid. Die Röntgenopazität ist eine wichtige klinische Eigenschaft von Füllungskompositen, die es dem Zahnarzt ermöglicht, z.B. Füllungen auf Röntgenaufnahmen zu erkennen.

Die erfindungsgemäßen Dentalwerkstoffe enthalten:
(1) 5 bis 40 Gew.-% und bevorzugt 5 bis 25 Gew.-% polyfunktionelle(s) radikalisch polymerisierbare(s) Monomer(e) (A),
(2) 2 bis 15 Gew.-% und bevorzugt 3 bis 10 Gew.-% monofunktionelle(s) radikalisch polymerisierbare(s) Monomer(e) (B),
(3) 0,1 bis 3,0 Gew.-% und bevorzugt 0,1 bis 2,0 Gew.-% Photoinitiator (C),
(4) 10 bis 85 Gew.-% und bevorzugt 40 bis 80 Gew.-% Füllstoff(e), und ggf.
(5) 0,1 bis 5,0 Gew.-%, vorzugsweise 0,1 bis 2,0 Gew.-% und ganz besonders bevorzugt 0,2 bis 1,5 Gew.-% Additiv(e).

Alle Angaben hierin beziehen sich auf die Gesamtmasse des Dentalwerkstoffs, wenn nicht anders angegeben. Die Füllstoffmenge Umfasst die Gesamtmenge aller Füllstoffe, wobei die Mengen der einzelnen Füllstoffkomponenten wie oben definiert sind.

Besonders bevorzugt sind solche Dentalwerkstoffe, welche aus den genannten Stoffen bestehen. Weiterhin sind solche Werkstoffe bevorzugt, bei denen die einzelnen Stoffe jeweils aus den oben genannten bevorzugten und besonders bevorzugten Stoffen ausgewählt sind.

Die erfindungsgemäßen Dentalwerkstoffe eignen sich besonders als dentale Zemente, Füllungskomposite, Beschichtungs- und Verblendmaterialien sowie als Materialien zur Herstellung von Inlays, Onlays, Kronen und Brücken, ganz besonders als sogenannte Bulk-Fill-Komposite. Unter Bulk-Fill-Kompositen werden dentalen Füllungsmaterialien verstanden, die selbst bei Schichtdicken von mehr als 3 mm, vorzugsweise mehr als 4 mm und insbesondere von 4-5 mm mit Licht gehärtet werden können. Sie erlauben die Herstellung auch großer Zahnfüllungen mit nur 1 bis 2 Schichten.

Die Dentalwerkstoffe eignen sich primär zur intraoralen Anwendung durch den Zahnarzt zur Restauration geschädigter Zähne (klinische Materialien). Sie können aber auch extraoral eingesetzt werden, beispielsweise bei der Herstellung oder Reparatur von Dentalrestaurationen (technische Materialien).

Die Erfindung wird im Folgenden anhand von Beispielen näher erläutert.

### Ausführungsbeispiele

### Beispiele 1 bis 3:

### Herstellung lichthärtender Kompositmaterialien

Es wurden Komposite mit den in Tabelle 1 beschriebenen Monomermischungen hergestellt. Die Zusammensetzungen der Komposite sind in Tabelle 2 angegeben. Die Komponenten wurden mit einem Kneter (Firma Linden) bzw. einem Mischer (Speedmixer, FlackTek Inc.) miteinander gemischt. Zur Bestimmung der Transluzenz der Komposite wurden jeweils gehärtete Prüfkörper (rund, Ø 20 mm, h = 1 mm) hergestellt und farbmetrisch mit Hilfe eines Spektralphotometers (Spectrophotometer CM-5, Minolta) vermessen. Die Polymerisation erfolgte mit einer LED-Lampe (Bluephase, Ivoclar Vivadent AG, 10 s bei 1000 mW/cm²). Die Messung von Biegefestigkeit und Durchhärtungstiefe erfolgte nach ISO 4049:2009: Zahnheilkunde - Polymerbasierende Restaurationsmaterialien. Der Polymerisationsschrumpf (Vol.-%) wurde nach ISO 17304:2013 bestimmt. Der Biegemodul wurde aus der Steigung der Messkurve für die Biegefestigkeit auf bekannte Weise berechnet. Die ermittelten Eigenschaften der Kompositpasten sind in Tabelle 3 zusammengestellt.

Das Beispiel 1 zeigt, dass gehärtete Komposite mit einer geringer Transluzenz (hohe Opazität) aus Kompositpasten mit hoher Transluzenz bei großer Durchhärtungstiefe hergestellt werden können. Das Beispiel 2 zeigt, dass der Effekt der Transluzenzabnahme bei der Polymerisation durch die Zugabe eines tiefbrechenden Glasfüllers (G018-090) deutlich verstärkt werden kann. Der Komposit in Beispiel 3 weist eine vorteilhafte Kombination von Eigenschaften auf.

**Tabelle 1: Zusammensetzung der Monomermischungen (Angaben in Gew.-%)**

| **Bestandteil** | **Nr. 1** | **Nr. 2** | **Nr. 3** |
|---|---|---|---|
| Ethoxyliertes p-Cumylphenolmethacrylat (CMP-1E) | 20 % | 20 % | 20 % |
| Bisphenol-A-Dimethacrylat mit 3 Ethoxygruppen (SR-348C)¹⁾ | 48,95 % | 11,8 % | 48,563 % |
| Bis-GMA | 20 % | 23, 8 % | 20 % |
| Urethandimethacrylat (UDMA) | - | 23,5 % | - |
| Bis(3-methacryloyloxymethyl)-tricylco[5.2.1.0^{2,6}]decan (TCP) | 10 % | - | 10 % |
| 2,2-Bis[4-(2-methacryloxypropoxy)-phenyl ] propan²⁾ | - | 20 % | - |
| Campherchinon | 0,2 % | 0,2 % | 0,2 % |
| (4-Dimethylamino)benzoesäureethylester (EMBO) | 0,8 % | 0,8 % | 0,8 % |
| Bis-(4-methoxybenzoyl)diethylgermanium³⁾ | 0,05 % | 0,1 % | 0,05 % |
| Stabilisator, Additive | - | - | 0,387 % |
| Brechungsindex der Mischung (ungehärtet) | 1,5416 | 1,5308 | 1,5416 |
| Brechungsindex der Mischung (gehärtet) | 1,5683 | 1,550 | 1,5683 |

| | | | |
|---|---|---|---|
| ¹⁾ CAS 41637-38-1 ²⁾ CAS 24447-72-1 ³⁾ CAS 1469766-31-1 (Ivocerin®, Ivoclar Vivadent AG) | | | |

**Tabelle 2: Zusammensetzung der Komposite (Angaben in Gew.-%)**

| **Bestandteil** | **Bsp 1** | **Bsp 2** | **Bsp 3** |
|---|---|---|---|
| Monomermischung 1 | 25 % | - | - |
| Monomermischung 2 | - | 30 % | - |
| Monomermischung 3 | - | - | 30 % |
| Bariumaluminiumsilicatglasfüller (Ø = 1,5 µm)¹⁾ | 75 % | 60 % | 18 % |
| Strontiumaluminiumsilicatglasfüller (Ø = 1,0 µm)²⁾ | - | 10 % | - |
| Bariumaluminiumsilicatglasfüller (Ø = 8 µm)¹⁾ | - | - | 36 % |
| Ytterbiumfluorid (YbF₃) | - | - | 4 % |
| Partikuläres Kompositmaterial (Ø = 30 µm)³⁾ | - | - | 12 % |

| | | | |
|---|---|---|---|
| ¹⁾ Dentalglas GM 27884 (Schott), Brechungsindex = 1,53 (Ø = mittlere Partikelgröße, Gewichtsmittel) ²⁾ Dentalglas G018-090 (Schott),Brechungsindex = 1,50 ³⁾ polymerisiertes, gemahlenes Kompositmaterial bestehend aus Monomermatrix, Glasfüller GM27884, und YbF₃; Brechungsindex = 1,53 | | | |

**Tabelle 3: Eigenschaften der Komposite**

| **Bsp.** | **Durchhärtungs tiefe** | **Transparenz** | | | **Biegefestigkeit** | **Biegemodul** | **ΔV_{P}¹⁾** |
|---|---|---|---|---|---|---|---|
| | | vor Polymerisation | 10 min nach Polymerisation | 24 h nach Polymerisation | | | |
| **1** | 6,8 mm²) | 46,3 % | 9,5 % | 9,2 % | - | - | - |
| **2** | 8,2 mm²) | 41,3 % | 10,7 % | - | - | - | - |
| **3** | 8,07 mm²) | 31 % | 10,07 % | 9,89 % | 117,2 MPa | 8,6 GPa | 3,48 % |

| | | | | | | | |
|---|---|---|---|---|---|---|---|
| ¹⁾ ΔV_{P} = Polymerisationsschrumpf gemäß ISO17304:2013 ²⁾ gemessener Wert | | | | | | | |

## Patentansprüche

1. Radikalisch polymerisierbarer Dentalwerkstoff, der
(1) 5 bis 40 Gew.-% mindestens eines polyfunktionellen radikalisch polymerisierbaren Monomers (A),
(2) 2 bis 15 Gew.-% mindestens eines monofunktionellen radikalisch polymerisierbaren Monomers (B),
(3) 0,1 bis 3,0 Gew.-% mindestens eines Photoinitiators für die radikalische Polymerisation (C) und
(4) 10 bis 85 Gew.-% Füllstoff(e) enthält,
**dadurch gekennzeichnet, dass** der Dentalwerkstoff einen Füllstoff (D) enthält und die Mischung der Monomere (A) und (B) einen Brechungsindex von 1,50 bis 1,70 aufweist und dass der Brechungsindex der Monomermischung vor der Härtung dem Brechungsindex des Füllstoffs (D) entspricht oder um bis zu 0,013 größer ist und nach der Härtung um mindestens 0,02 größer als der Brechungsindex des Füllstoffs (D) ist, und
der Dentalwerkstoff bezogen auf die gesamte Füllstoffmenge
- 0 bis 15 Gew.-% Füllstoff (E), dessen Brechungsindex um 0,02 bis maximal 0,155 kleiner als der Brechungsindex der Mischung der Monomere (A) und (B),
- 0 bis 10 Gew.-% Füllstoff, der sichtbares Licht nicht streut und die Transluzenz nicht beeinflusst und
- maximal 15,5 Gew.-% Füllstoff (F) enthält, der in keine dieser Kategorien fällt.

2. Dentalwerkstoff nach Anspruch 1, der als Komponente (B) Methacrylat, Methyl-, Ethyl-, 2-Hydroxyethyl-, Butyl-, Benzyl-, Tetrahydrofurfuryl- oder Isobornylmethacrylat, p-Cumyl-phenoxyethylenglycolmethacrylat (CMP-1E) oder eine Mischung davon enthält.

3. Dentalwerkstoff nach Anspruch 1 oder 2, der als Komponente (A) mindestens ein polyfunktionelles Methacrylat, Bisphenol-A-dimethacrylat, 2,2-Bis[4-(2-hydroxy-3-methacryloyloxypropyl)phenyl]propan (Bis-GMA; ein Additionsprodukt aus Methacrylsäure und Bisphenol-A-diglycidylether), ethoxy- oder propoxyliertes Bisphenol-A-Dimethacrylat, 2-[4-(2-Methacryloyloxyethoxyethoxy)phenyl]-2-[4-(2-methacryloyloxyethoxy)phenyl]propan), 2,2-Bis[4-(2-methacryloxypropoxy)phenyl]propan, 1,6-Bis-[2-methacryloyloxyethoxycarbonylamino]-2,2,4-trimethylhexan (UDMA; ein Additionsprodukt aus 2-Hydroxyethylmethacrylat und 2,2,4-Trimethylhexamethylendiisocyanat), Di-, Tri- oder Tetraethylenglycoldimethacrylat, Trimethylolpropantrimethacrylat, Pentaerythrittetramethacrylat, Glycerindi- oder -trimethacrylat, 1,4-Butandioldimethacrylat, 1,10-Decandioldimethacrylat (D₃MA), 1,12-Dodecandioldimethacrylat oder Bis(3-methacryloyloxymethyl)tricyclo-[5.2.1.0^{2,6}]decan oder eine Mischung davon enthält.

4. Dentalwerkstoff nach einem der vorangehenden Ansprüche, der als Komponente (A) und/oder Komponente (B) ausschließlich Monomere mit einem Brechungsindex von 1,50 bis 1,70 enthält.

5. Dentalwerkstoff nach einem der vorangehenden Ansprüche, der mindestens ein schwerflüchtiges Monomethacrylat, vorzugsweise in einer Menge von 5-30 Gew.-%, mindestens ein hochviskoses difunktionelles Methacrylat, vorzugsweise in einer Menge von 5-50 Gew.-%, und mindestens ein niedrigviskoses Dimethacrylat, vorzugsweise in einer Menge von 5-30 Gew.-%, enthält, wobei sich die Prozentangaben jeweils auf die Gesamtmasse der Monomermischung beziehen.

6. Dentalwerkstoff nach einem der vorangehenden Ansprüche, der einen Füllstoff (E) enthält, dessen Brechungsindex um 0,03 bis 0,055 kleiner als der Brechungsindex der Mischung der Monomere (A) und (B) ist.

7. Dentalwerkstoff nach Anspruch 6, bei dem der Anteil des Füllstoffs (E) an der gesamten Füllstoffmenge 1 bis 5 Gew.-% beträgt.

8. Dentalwerkstoff nach einem der vorangehenden Ansprüche, der 0 bis 9,7 Gew.-% bezogen auf die Gesamtfüllstoffmenge an Füllstoff (F) enthält, der in keine dieser Kategorien fällt.

9. Dentalwerkstoff nach Anspruch 8, bei dem der Brechungsindex des weiteren Füllstoffs (F) um maximal 0,055 größer als der Brechungsindex der Mischung der Monomere (A) und (B) ist.

10. Dentalwerkstoff nach einem der vorangehenden Ansprüche, der als Füllstoff ein pulverförmiges röntgenopakes Glas mit einer durchschnittlichen Teilchengröße von 0,01 bis 15 µm, einen röntgenopaken Füllstoff, Ytterbiumtrifluorid, ein Mischoxid aus SiO₂, ZrO₂, ZnO und/oder TiO₂, nanopartikuläres Tantal(V)-oxid, Bariumsulfat, ein Mischoxid von SiO₂ mit Ytterbium(III)-oxid oder Tantal(V)-oxid, pyrogene Kieselsäure oder Fällungskieselsäure, ein organisches Polymerpulver, organisch-anorganischen Füllstoff oder eine Mischung davon enthält.

11. Dentalwerkstoff nach einem der vorangehenden Ansprüche, der als Komponente (C)
- ein α-Diketon, 9,10-Phenanthrenchinon, 1-Phenylpropan-1,2-dion, Diacetyl oder 4,4'-Dichlorbenzil oder ein Derivat davon, Campherchinon (CC) oder ein Derivat davon, ggf. in Kombination mit einem Beschleuniger, oder eine Mischung davon und/oder
- eine Acyl- oder Bisacylgermanium-Verbindung und/oder
- ein Acyl- oder Bisacylphosphinoxid enthält.

12. Dentalwerkstoff nach einem der vorangehenden Ansprüche, der als weitere Komponente mindestens ein Additiv enthält, das aus Stabilisatoren, Polymerisationsstabilisatoren, Farbmitteln, antibakteriellen Wirkstoffen, fluoridionenabgebenden Additiven, optischen Aufhellern, Fluoreszenzmitteln, UV-Absorbern, Stoffen zur Verbesserung der Bruchzähigkeit und/oder Effektmittel ausgewählt ist.

13. Dentalwerkstoff nach einem der vorangehenden Ansprüche, der
(1) 5 bis 25 Gew.-% polyfunktionelle(s) radikalisch polymerisierbare(s) Monomer(e) (A),
(2) 3 bis 10 Gew.-% monofunktionelle(s) radikalisch polymerisierbare(s) Monomer(e) (B),
(3) 0,1 bis 2,0 Gew.-% Photoinitiator (C),
(4) 40 bis 80 Gew.-% Füllstoff(e) und ggf.
(5) 0,2 bis 1,5 Gew.-% Additiv(e) enthält.

14. Dentalwerkstoff gemäß einem der vorhergehenden Ansprüche zur Verwendung als dentaler Zement, Füllungskomposit, Beschichtungsmaterial, Verblendmaterial oder Bulk-Fill-Komposit.

15. Verwendung eines Dentalwerkstoffs gemäß einem der Ansprüche 1 bis 13 zur Herstellung von Inlays, Onlays, Kronen oder Brücken.

## Claims

1. Radically polymerisable dental material comprising
(1) 5 to 40 wt % of at least one polyfunctional radically polymerisable monomer (A),
(2) 2 to 15 wt % of at least one monofunctional radically polymerisable monomer (B),
(3) 0.1 to 3.0 wt.% of at least one photo initiator for the radical polymerisation (C) and
(4) 10 to 85 wt % filler(s),
**characterised in that** the dental material comprises a filler (D) and the refractive index of the mixture of monomers (A) and (B) is from 1.50 to 1.70 and that the refractive index of the monomer mixture before curing corresponds to the refractive index of the filler (D) or is greater by up to 0.013 and after curing is higher than the refractive index of the filler (D) by at least 0.02, and
the dental material, relative to the total quantity of filler, comprises
- 0 to 15 wt % filler (E), the refractive index of which is 0.02 to at most 0.155 less than the refractive index of the mixture of monomers (A) and (B),
- 0 to 10 wt % filler that does not scatter visible light and does not affect the translucence and
- at most 15.5 wt % filler (F) that does not fall within any of these categories.

2. Dental material according to claim 1 comprising as the component (B) methyl, ethyl, 2-hydroxyethyl, butyl, benzyl, tetrahydrofurfuryl or *iso*bornyl methacrylate, *p*-cumyl-phenoxyethylene glycol methacrylate (CMP-1E) or a mixture thereof.

3. Dental material according to claim 1 or 2 comprising as the component (A) at least one polyfunctional methacrylate, bisphenol-A-dimethacrylate, 2,2-bis[4-(2-hydroxy-3-methacryloyloxypropyl)phenyl]propane (bis-GMA; an addition product of methacrylic acid and bisphenol-A-diglycidyl ether), ethoxylated or propoxylated bisphenol-A-dimethacrylate, 2-[4-(2-methacryloyloxyethoxyethoxy)phenyl]-2-[4-(2-methacryloyloxyethoxy)phenyl]-propane), 2,2-bis[4-(2-methacryloxypropoxy)phenyl]propane, 1,6-bis-[2-methacryloyloxyethoxycarbonylamino]-2,2,4-trimethylhexane (UDMA; an addition product of 2-hydroxyethyl methacrylate and 2,2,4-trimethylhexamethylene diisocyanate), di-, tri- or tetraethylene glycol dimethacrylate, trimethylolpropane trimethacrylate, pentaerythritol tetramethacrylate, glycerol di- or trimethacrylate, 1,4-butanediol dimethacrylate, 1,10-decanediol dimethacrylate (D₃MA), 1,12-dodecanediol dimethacrylate or bis-(3-methacryloyloxymethyl)tricyclo-[5.2.1.0^{2,6}]decane or a mixture thereof.

4. Dental material according to one of the preceding claims comprising exclusively monomers with a refractive index of from 1.50 to 1.70 as the component (A) and/or component (B).

5. Dental material according to one of the preceding claims comprising at least one low-volatile monomethacrylate, preferably in a quantity of 5-30 wt %, at least one highly viscous difunctional methacrylate, preferably in a quantity of 5-50 wt %, and at least one low-viscosity dimethacrylate, preferably in a quantity of 5-30 wt %, wherein the percentages in each case refer to the total weight of the monomer mixture.

6. Dental material according to one of the preceding claims comprising filler (E), the refractive index of which is lower than the refractive index of the mixture of the monomers (A) and (B) by 0.03 to 0.055.

7. Dental material according to claim 6, in which the proportion of the filler (E) in the total amount of filler is 1 to 5 wt %.

8. Dental material according to one of the preceding claims comprising, relative to the total filler quantity, 0 to 9.7 wt % of filler (F) that does not fall within any of these categories.

9. Dental material according to claim 8, in which the refractive index of the additional filler (F) is greater than the refractive index of the mixture of monomers (A) and (B) by 0.055 at most.

10. Dental material according to one of the preceding claims comprising as the filler a pulverulent X-ray opaque glass with an average particle size of 0.01 to 15 µm, an X-ray opaque filler, ytterbium trifluoride, a mixed oxide of SiO₂, ZrO₂, ZnO and/or TiO₂, nanoparticulate tantalum (V) oxide, barium sulfate, a mixed oxide of SiO₂ with ytterbium (III) oxide or tantalum (IV) oxide, pyrogenic silica or precipitated silica, an organic polymer powder, organic-inorganic filler or a mixture thereof.

11. Dental material according to one of the preceding claims comprising as the component (C)
- an α-diketone, 9,10-phenanthrenequinone, 1-phenylpropane-1,2-dione, diacetyl or 4,4'-dichlorobenzil or a derivative thereof, camphorquinone (CQ) or a derivative thereof, optionally in combination with an accelerator, or a mixture thereof and/or
- an acyl or bisacyl germanium compound and/or
- an acyl or bisacyl phosphine oxide.

12. Dental material according to one of the previous claims comprising as a further component at least one additive that is selected from stabilisers, polymerisation stabilisers, dyes, antibacterial active ingredients, fluoride ion-releasing additives, optical brighteners, fluorescent agents, UV absorbers, substances for improving the fracture toughness and/or effect agents.

13. Dental material according to one of the preceding claims comprising
(1) 5 to 25 wt % polyfunctional radically polymerisable monomer(s) (A),
(2) 3 to 10 wt % monofunctional radically polymerisable monomer(s) (B),
(3) 0.1 to 2.0 wt % photo initiator (C),
(4) 40 to 80 wt % filler(s) and optionally
(5) 0.2 to 1.5 wt % additive(s).

14. Dental material according to one of the previous claims for use as a dental cement, filling composite, coating material, veneering material or bulk fill composite.

15. Use of a dental material according to one of claims 1 to 13 for manufacturing inlays, onlays, crowns or bridges.

## Revendications

1. Matériau dentaire polymérisable par voie radicalaire, contenant
1) de 5 à 40 % en poids d'au moins un monomère (A), polyfonctionnel et polymérisable par voie radicalaire,
2) de 2 à 15 % en poids d'au moins un monomère (B), monofonctionnel et polymérisable par voie radicalaire,
3) de 0,1 à 3,0 % en poids d'au moins un photoamorceur (C) pour polymérisation par voie radicalaire,
4) et de 10 à 85 % en poids de charge(s),
**caractérisé en ce que** ce matériau dentaire contient une charge (D), que le mélange des monomères (A) et (B) présente un indice de réfraction de 1,50 à 1,70, et que l'indice de réfraction du mélange de monomères, avant durcissement, correspond à l'indice de réfraction de la charge (D) ou lui est supérieur d'au plus 0,013, mais après durcissement, est supérieur d'au moins 0,02 à l'indice de réfraction de la charge (D),
et **en ce que** ce matériau dentaire contient, par rapport à la quantité totale de charges,
- de 0 à 15 % en poids de charge (E) dont l'indice de réfraction est inférieur, de 0,02 à au plus 0,155, à l'indice de réfraction du mélange des monomères (A) et (B),
- de 0 à 10 % en poids de charge qui ne disperse pas la lumière visible et qui n'influence pas la translucidité,
- et au plus 15,5 % en poids de charge (F) qui n'entre dans aucune de ces catégories.

2. Matériau dentaire conforme à la revendication 1, qui contient, en tant que composant (B), un méthacrylate, du méthacrylate de méthyle, d'éthyle, de 2-hydroxy-éthyle, de butyle, de benzyle, de tétrahydrofuryle ou d'isobornyle, du méthacrylate de cumyl-phénoxyethylèneglycol (CMP-1E), ou un mélange de ceux-ci.

3. Matériau dentaire conforme à la revendication 1 ou 2, qui contient, en tant que composant (A), au moins un méthacrylate polyfonctionnel, du diméthacrylate de bisphénol A, du 2,2-bis[4-(2-hydroxy-3-méthacryloyloxy-propyl)-phényl]-propane (ou bis-GMA, un produit d'addition d'acide méthacrylique et d'éther diglycidylique de bisphénol A), du diméthacrylate de bisphénol A éthoxylé ou propoxylé, du 2-[4-(2-méthacryloyloxyéthoxy-éthoxy)-phényl]-2-[4-(2-méthacryloyloxy-éthoxy)-phényl]-propane, du 2,2-bis[4-(2-méthacryloyloxy-propoxy)-phényl]-propane, du 1,6-bis(2-méthacryloyloxy-éthoxy-carbonylamino)-2,2,4-triméthyl-hexane (ou UDMA, un produit d'addition de méthacrylate de 2-hydroxy-éthyle et de 2,2,4-triméthyl-hexaméthylènediisocyanate), du diméthacrylate de diéthylène-glycol, de triéthylèneglycol ou de tétraéthylène-glycol, du triméthacrylate de triméthylolpropane, du tétraméthacrylate de pentaérythritol, du diméthacrylate ou du triméthacrylate de glycérol, du diméthacrylate de butane-1,4-diol, du diméthacrylate de décane-1,10-diol (ou D₃MA), du diméthacrylate de dodécane-1,12-diol, ou du bis(3-méthacryloyloxyméthyl)-tricyclo-[5.2.1.0^{2,6}]décane, ou un mélange de ces composés.

4. Matériau dentaire conforme à l'une des revendications précédentes, qui contient exclusivement, en tant que composant(s) (A) et/ou composant(s) (B), des monomères dont l'indice de réfraction vaut de 1,50 à 1,70.

5. Matériau dentaire conforme à l'une des revendications précédentes, qui contient au moins un monométhacrylate peu volatil, de préférence en une proportion de 5 à 30 % en poids, au moins un méthacrylate difonctionnel fortement visqueux, de préférence en une proportion de 5 à 50 % en poids, et au moins un diméthacrylate faiblement visqueux, de préférence en une proportion de 5 à 30 % en poids, étant entendu que chacun de ces pourcentages est rapporté à la masse totale du mélange de monomères.

6. Matériau dentaire conforme à l'une des revendications précédentes, qui contient une charge (E) dont l'indice de réfraction est inférieur de 0,03 à 0,055 à l'indice de réfraction du mélange des monomères (A) et (B).

7. Matériau dentaire conforme à la revendication 6, dans lequel la proportion de la charge (E), rapportée à la quantité totale de charges, vaut de 1 à 5 % en poids.

8. Matériau dentaire conforme à l'une des revendications précédentes, qui contient de 0 à 9,7 % en poids, par rapport à la quantité totale de charges, de charge (F) qui n'entre dans aucune des autres catégories.

9. Matériau dentaire conforme à la revendication 8, dans lequel l'indice de réfraction de la charge supplémentaire (F) est supérieur d'au plus 0,055 à l'indice de réfraction du mélange des monomères (A) et (B).

10. Matériau dentaire conforme à l'une des revendications précédentes, qui contient, en tant que charge, un verre opaque aux rayons X, sous forme de poudre dont les particules ont une taille moyenne de 0,01 à 15 µm, une charge opaque aux rayons X, du trifluorure d'ytterbium, un oxyde mixte à base de SiO₂, ZrO₂, ZnO et/ou TiO₂, de l'oxyde de tantale-V en nanoparticules, du sulfate de baryum, un oxyde mixte à base de silice et d'oxyde d'ytterbium-III ou d'oxyde de tantale-V, de la silice pyrogénée ou de la silice précipitée, un polymère organique en poudre, une charge organique-inorganique, ou un mélange de telles charges.

11. Matériau dentaire conforme à l'une des revendications précédentes, qui contient, en tant que composant (C),
- une α-dicétone, de la 9,10-phénanthrène-quinone, de la 1-phénylpropane-1,2-dione, du diacétyle ou du 4,4'-dichloro-benzile ou un dérivé de l'un de ces composés, de la camphoquinone (CC) ou un dérivé de celle-ci, éventuellement en combinaison avec un accélérateur, ou un mélange de tels composés,
- et/ou un composé de type acyl-germanium ou bis(acyl)-germanium,
- et/ou un oxyde d'acyl-phosphine ou de bis(acyl)-phosphine.

12. Matériau dentaire conforme à l'une des revendications précédentes, qui contient, en tant qu'autre(s) composant(s), au moins un adjuvant choisi parmi les suivants : stabilisants, stabilisants de polymérisation, colorants, agents antibactériens, adjuvants libérant des ions fluorure, azurants optiques, agents de fluorescence, absorbeurs UV, substances améliorant la résistance à la rupture et/ou agents actifs.

13. Matériau dentaire conforme à l'une des revendications précédentes, qui contient
1) de 5 à 25 % en poids de monomère(s) (A), polyfonctionnel(s) et polymérisable(s) par voie radicalaire,
2) de 3 à 10 % en poids de monomère(s) (B), monofonctionnel(s) et polymérisable(s) par voie radicalaire,
3) de 0,1 à 2,0 % en poids de photoamorceur (C),
4) et de 10 à 80 % en poids de charge(s),
5) et en option, de 0,2 à 1,5 % en poids d'adjuvant(s).

14. Matériau dentaire conforme à l'une des revendications précédentes, pour utilisation en tant que ciment dentaire, composite d'obturation, matériau de revêtement, matériau de revêtement de couleur naturelle ou composite d'obturation « bulk-fill ».

15. Utilisation d'un matériau dentaire conforme à l'une des revendications 1 à 13 pour la fabrication d'incrustations « in-lay » ou « on-lay », de couronnes ou de bridges.
